# EUROPÄISCHE PATENTSCHRIFT

(11) **EP 0 674 888 B1**
(45) Veröffentlichungstag und Bekanntmachung des Hinweises auf die Patenterteilung: **03.05.2000**
(21) Anmeldenummer: 95102744.0
(22) Anmeldetag: 27.02.1995
(51) Int. Cl.: A61F 2/46, B01F 13/00, B01F 13/06, B65D 81/32

(54) **Mischvorrichtung**
Mixing device
Dispositif de mélange

(30) Priorität: 21.03.1994 DE 4409610
(43) Veröffentlichungstag der Anmeldung: 04.10.1995
(73) Patentinhaber: Scandimed International AB, 275 37 Sjöbo (SE)
(72) Erfinder: Lidgren, Lars Ake Alvar, S-227 31 Lund (SE); Bauer, Hans Jörg, D-55234 Flomborn (DE)
(74) Vertreter: Wagner, Karl Heinz

(56) Entgegenhaltungen:
- EP-A- 0 194 508
- WO-A-93/22041
- DE-A- 4 302 230
- DE-B- 1 939 316
- FR-B- 2 447 875
- US-A- 4 159 570
- US-A- 4 463 875
- US-A- 4 676 655

## Beschreibung

Die vorliegende Erfindung bezieht sich auf eine Mischvorrichtung zum Mischen von zumindest zwei Stoffen, vorzugsweise flüssiger Monomer und pulverförmiger Polymer, zum Herstellen von Knochenzement. Diese Mischvorrichtung geht näher aus dem Oberbegriff des nachfolgenden Patentanspruchs 1 hervor.

Die Implantationstechnik in der medizinischen Heilkunde hat in den letzten Jahren erhebliche Fortschritte erzielt. Speziell die Applikationsmethoden für Knochenzemente bei der Implantation von Prothesen bzw. die Verwendung von Knochenzement als Platzhalter in der restaurativen Chirurgie erreichten erstaunliche Fortschritte.

In der Ursprungszeit der Knochenzemente wurden diese unter normalen atmosphärischen Bedingungen in einem Becher angerührt, mit der Hand geknetet, zu "Würsten" geformt und in den zu füllenden Defekt eingeführt und per Hand verdichtet. Diese Methode hat sich lange Zeit als die einzig mögliche Applikationsmethode für Knochenzemente gehalten.

Daran anschliessend wurde eine Verbesserung in Form einer Applikation über eine Kartusche entwickelt. In dieser Ära wurde der Zement ebenfalls unter normalen atmosphärischen Bedingungen angerührt, dann aber in eine Kartusche umgefüllt und durch diese mittels eines mechanischen Kraftübertragungsapparates (Pistole) aus der Kartuschenmündung herausgepresst.

Der nächste Fortschritt in der Applikationsmethode war die vollständige Elimination eines Handkontaktes mit dem Zement, indem das Anrühren in einem speziellen Becher erfolgte, von dem aus der angerührte Zement direkt in die Kartusche eingeführt werden konnte.

In diesen Zeiten war die Lebensdauer von implantierten Prothesen überwiegend vom Geschick der Operateure abhängig. Die Lebenserwartung der Implantate richtete sich nach der Perfektion, mit der der Zement angerührt und appliziert wurde. Die Lebenserwartung hing kaum von der Qualität der Prothese ab (bis auf die bekannten Schaftbrüche), da Knochenzement in seiner Lebenserwartung weit unter der der Prothesen lag. Hier erkannten die Mediziner und Forscher ein wesentliches Entwicklungsdefizit und erforschten die grundlegenden Ursachen für das Versagen der Knochenzemente. Im wesentlichen ergaben sich zwei Hauptursachen für die relativ kurze Lebenserwartung der Knochenzemente. Zum einen war es eine nicht vollständige Mischung der Monomere mit den Polymeren, was zu geringen und unterschiedlichen Primärfestigkeiten innerhalb des Zementmantels um die Prothesen führte und was durch nicht gebundene und auspolymerisierte Monomer- und Polymeranteile zu toxikologischen Problemen und entzündlichen Gewebereaktionen führte. Zum zweiten ergaben sich bei den Untersuchungen mechanische Inhomogenitäten in dem Zement, hervorgerufen durch eingeschlossene Luftblasen, welche die Dauerschwingfestigkeit der Knochenzemente erheblich nachteilig beeinflussen. Aber auch grobe Defekte durch eingeschlossene Luftblasen bewirkten einen vorzeitigen Bruch des Zementmantels.

Nach der weitestgehenden Verminderung oder Elimination von Infektionen durch die Integration von antibakteriellen Wirkstoffen in den Zementen, die ihrerseits die Lebenserwartung zementierter Prothesen verlängerten, ergab sich klar die Notwendigkeit, auch die mechanischen Eigenschaften der Knochenzemente zu verbessern, ebenfalls mit dem Ziel längerer Prothesenstandzeiten.

Diesen Erkenntnissen Rechnung tragend, entwickelte L. Lidgren eine Methode zur Anmischung von Knochenzementen unter gleichzeitiger Reduzierung der Porenanteile. Funktionell war dies die Anmischung der Knochenzemente unter Vakuum in der Applikationskartusche. So wird das Monomer in die Kartusche eingefüllt, das Polymerpulver aufgeschüttet und die Kartusche mit einer Mischeinrichtung verschlossen. Nach Anlegen eines Vakuums wird der Zement nun mit dem Monomer unter Vakuum gemischt. Es folgt eine Quellzeit, ebenfalls unter Vakuum, und erst dann wird der Knochenzement mittels einer Pistole aus der Kartusche über einen Schnorchel appliziert. Dieses System der Zementanmischung und Applikation ist unter dem Namen "Optivac" der Firma MIT AB in Handel und entsprechende Studien haben die Effektivität und die Sicherheit dieses Systems belegt.

Experimentell konnte bewiesen werden, das durch einen derartigen Mischvorgang die Porosität (Anzahl und Grösse der Lufteinschlüsse) der Zementmatrix deutlich verringert und infolgedessen die mechanische Festigkeit eines so angemischten Zementes wesentlich verbessert werden kann.

Eine Literaturübersicht bezüglich der oben erwähnten Mischung von Knochenzementen unter Vakuum folgt am Schluss dieser Beschreibung.

Aus der Patentliteratur seien die US-Patentschriften 4 463 875 und 4 973 168, die europäische Patentanmeldung EP 0 194 508 sowie die deutsche Patentanmeldung DE 43 02 230 erwähnt.

Die US-Patentschrift 4 463 875 beschreibt eine Mischvorrichtung, wobei zwei zu mischende Stoffe im voraus getrennt voneinander in der Mischvorrichtung verpackt sind. Die Stoffe werden miteinander in Kontakt gebracht und dann gemischt. Um dies durchzuführen, wird die Verpackung von aussen mit hin- und hergehenden Bewegungen belastet, so dass sie sich verformt und dadurch die beiden Stoffe mischt. Nachteilig hierbei ist, dass mit diesen Mischbewegungen kein Knochenzement mit erstrebter Qualität erreicht wurde. Ausserdem ist eine komplizierte Behälterausführung erforderlich, um diese Mischbewegungen ausführen zu können.

Die US-Patentschrift 4 973 168 beschreibt eine Mischvorrichtung, in der der eine Stoff von den zu mischenden Stoffen im voraus in der Mischvorrichtung verpackt ist, während der andere Stoff unabhängig von der Mischvorrichtung in einem separaten Behälter verpackt ist. Zum Mischen der beiden Stoffe wird der Behälter in die Mischvorrichtung entleert, wonach der Stoff des Behälters mit dem im voraus in der Mischvorrichtung verpackten Stoff durch ein inneres Mischorgan gemischt wird. Nachteilig hierbei ist, dass ein separater Behälter für den einen Stoff notwendig ist und dass das Risiko vorliegt, dass nämlich der separate Behälter nicht vollständig entleert wird, oder dass ein Teil des Stoffes des separaten Behälters verschüttet wird und der erhaltene Knochenzement deshalb nicht die gestellten Qualitätsforderungen erfüllt.

Die europäische Patentanmeldung EP 0 194 508 beschreibt eine Mischvorrichtung für die Mischung anderer Mittel, wobei kein Vakuum erzeugt wird.

Die weiterhin erwähnte Patentanmeldung DE 43 02 230 beschreibt eine Mischvorrichtung in die die beiden Stoffe offen hineingeschüttet werden. Bei diesem Verfahren ist die Vorbereitung vor der Mischung zeitraubend, es können Vorbereitungsfehler vor der Mischung vorkommen und es können gefährliche Gase entstehen die die Umgebung belasten.

Die vorliegende Erfindung beabsichtigt, eine Mischvorrichtung zu schaffen, die es ermöglicht, die bisherige Mischtechnik zu verbessern und auch Vorbereitungsfehler vor der Mischung weitgehend auszuschliessen.

Dies wird gemäss der Erfindung dadurch erreicht, dass die Mischvorrichtung die im Anspruch 1 definierten Merkmale aufweist.

Durch diese Merkmale wird insbesondere erreicht, dass keine Umfüllung der Stoffe notwendig ist, und ein Verschütten zumindest des einen Stoffes ausgeschlossen wird, und dass die Stoffe ausserdem genügend effektiv gemischt werden können, um einen Knochenzement mit der erstrebten Qualität zu erhalten.

Hierdurch können auch die Zementkomponenten steril ohne zusätzliche Handgriffe bereits zur Anmischung im Mischbehälter vorliegen, was u.a. zu einer Reduzierung der technischen Handgriffe und Minimierung der Umweltgefahr und der Abfallmengen führt. Ausserdem wird das Kontaminationsrisiko durch den Knochenzement reduziert und es entstehen keine zusätzlichen Abfallmengen. Auch werden Fehler bei der Reihenfolge des Anmischvorgangs ausgeschlossen.

Die Mischvorrichtung gemäss der Erfindung wird in der folgenden Beschreibung mit Hinweis auf die nachfolgenden Zeichnungen näher beschrieben, wobei zeigt:
- Fig. 1: schematisch einen Mischbehälter gemäss der Erfindung mit einer Seitenansicht, wobei zwei Stoffe getrennt von einander in zwei Behältern angeordnet sind;
- Fig. 2: schematisch den Mischbehälter gemäss Fig. 1 beim Öffnen einer Verbindung zwischen den zwei Behältern, wobei in dem einen Behälter Vakuum erzeugt wird;
- Fig. 3: schematisch den Mischbehälter gemäss Fig. 1 beim Mischen der Stoffes in einem Mischraum in dem Vakuum herrscht;
- Fig. 4: schematisch den Mischbehälter gemäss Fig. 1 beim Aufsammeln unter Vakuum des aus den Stoffen bestehenden Knochenzementes in dem Mischraum;
- Fig. 5: schematisch den Mischbehälter gemäss Fig. 1 mit daran angeschlossenen Entleerrohr;
- Fig. 6: schematisch den Mischbehälter gemäss Fig. 1 beim Entleeren des Knochenzements mit einer Druckvorrichtung; und

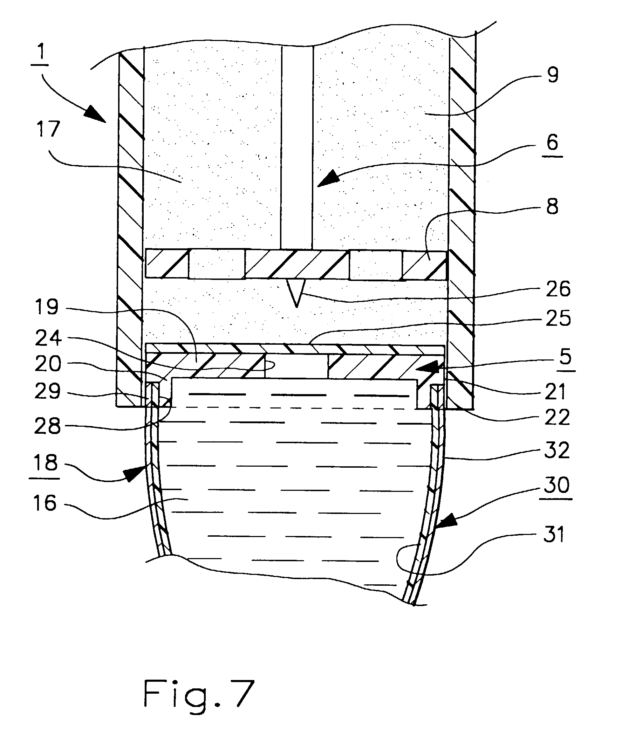

- Fig. 7: einen vergrösserten Schnitt von einen Teil des Mischbehälters gemäss Fig. 1.

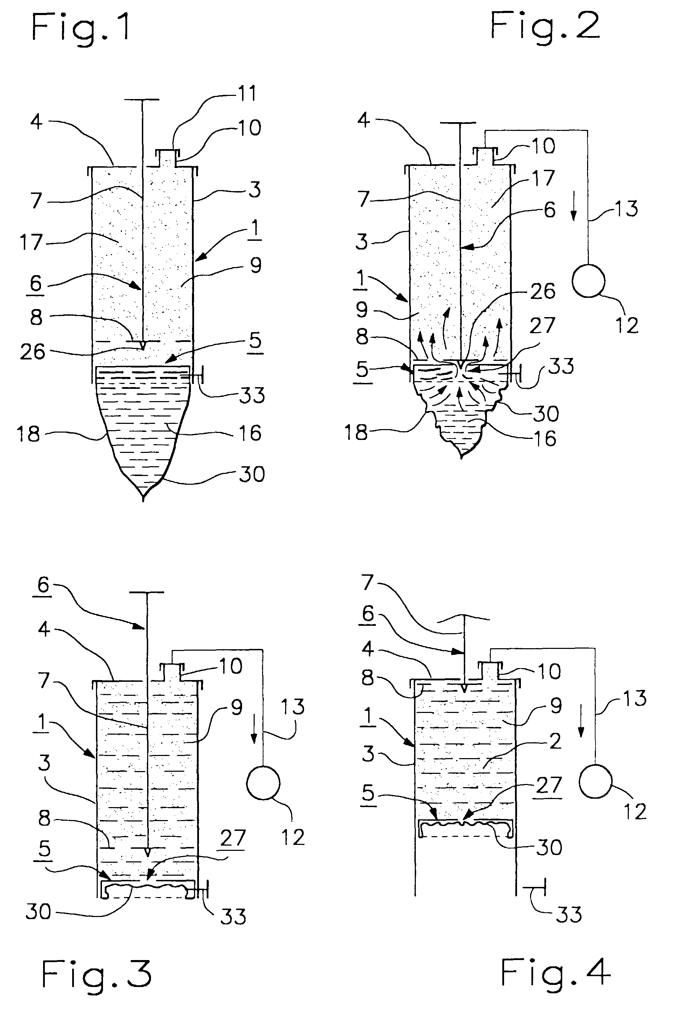

Der in Fig. 1 gezeigte Mischbehälter 1 hat die Form einer Kartusche, in der Knochenzement 2 fertiggestellt und mit dieser appliziert wird. Dieser Mischbehälter 1 besteht vorzugsweise aus einem zylindrischen Behälter 3, der an der einen Seite mit einem Deckel 4 und an der entgegengesetzten Seite mit einem verschiebbaren an Kolben 5 dicht verschlossen ist. Ein Mischorgan 6 besteht aus einem Griff 7 und einem daran befestigten Rührorgan 8, das sich in einem Mischraum 9 befindet. Der Griff 7 ist langgestreckt und verschiebbar und vorzugsweise auch drehbar im Deckel 4 gelagert, so dass er von aussen verschoben und gedreht werden kann, um das Rührorgan 8 im Mischraum 9 hin und her bewegen zu können und vorzugsweise zu drehen, um im Mischraum 9 befindliche Stoffe zu mischen.

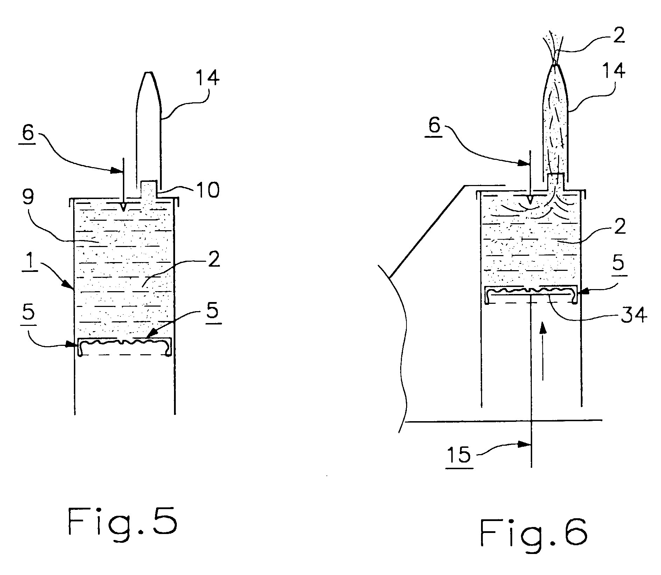

Der Deckel 4 hat ein Anschlussrohr 10, das mit einem abnehmbaren Verschluss 11 verschliessbar ist. An der Anschlussrohr 10 kann eine vakuumerzeugende Vorrichtung 12 durch einen Schlauch 13 angeschlossen werden (siehe Fig. 2), um in dem Mischraum 9 partielles Vakuum, vorzugsweise 60 - 95 % Vakuum, zu erzeugen. Ausserdem kann an dem Anschlussrohr 10 ein Entleerrohr 14 befestigt werden (siehe Fig. 5 und 6), wodurch fertiggestellter Knochenzement 2 aus dem Mischraum 9 entleert wird, um diesen zu applizieren. Hierbei wird der Knochenzement 2 mit dem Kolben 5 aus dem Mischraum 9 gepresst, wobei der Kolben 5 mit einer Druckvorrichtung 15, z.B. einer Druckpistole, verschoben wird (siehe Fig. 6).

In dem Mischraum 9 ist der eine Stoff 17, vorzugsweise Pulverförmiger Polymer, angeordnet und in einem Behälter 18 ist getrennt davon ein anderer Stoff 16, vorzugsweise flüssiger Monomer, angeordnet. Diese Stoffe 16, 17 werden miteinander in Kontakt gebracht und gemischt um Knochenzement 2 davon herzustellen.

Der Kolben 5 besteht aus einer Wand 19 und einem hülsenförmigen Teil 20 der von der Wand 19 in Richtung auswärts im Verhältnis zum Mischraum 9 verläuft. Die Aussenseite 21 der Wand 19 und des Teils 20 liegt dichtend und verschiebbar gegen die Innenseite 22 des zylindrischen Behälters 3 an. Im Zentrum der Wand 19 ist eine Verbindungsöffnung 24 angeordnet die durch einen Membran 25 abgeschlossen wird. Dieser Membran 25 wird mit einem Öffner 26 geöffnet, z.B. mit einem Dorn 26 am Rührorgan 8 durchstochen, wodurch die Wand 19 geöffnet wird und dadurch eine Verbindung 27 zwischen dem Inneren des Behälters 18 und dem Mischraum 9 zustande kommt.

Der hülsenförmige Teil 20 kann zylindrische Form aufweisen und an der Aussenseite 21 eine rund um denselben verlaufende Nute 28. In diese Nute 28 greift ein hülsenförmiger Endteil 29 des Behälters 18 ein und dieser Endteil 29 umgibt den hülsenförmigen Teil 20 des Kolbens 5 und ist dichtend an diesen angeordnet.

Die Wände 30 des Behälters 18 bestehen bespielsweise aus mehreren Schichten, wobei eine innere Schicht 31 aus Kunststoff, z.B. Polyäthylen, besteht und eine äussere Schicht 32 aus Metallmaterial, z.B. Aluminium, besteht.

Die innere Schicht 31 liegt gegen den hülsenförmigen Teil 20 des Kolbens 5 an, welcher Teil 20 vorzugsweise aus demselben oder ähnlichen Kunststoffmaterial, z.B. Polyäthylen, wie die Schicht 31 besteht. Das Kunststoffmaterial des Kolbens 5 und der inneren Schicht 31 des Behälters 18 können mit einander durch Wärmebehandlung verschmolzen sein, wobei die Wärme z.B. durch s.g. Ultrafrequenzschweissen erreicht werden kann.

Um die Mischoperation durchzuführen, wird der Mischraum 9 des Mischbehälters 1, vorzugsweise an dem Platz, z.B. in einem Operationsraum, wo die Mischung durchgeführt werden woll, an die vakuumerzeugende Vorrichtung 12 angeschlossen, wobei im Mischraum 9 partielles Vakuum erzeugt wird. Danach wird das Mischorgan 6 als Öffner 26 benutzt, wobei der Dorn 26 durch den Membran 25 gedrückt wird. Durch die hierbei hergestellte Verbindung 27 wirkt sofort das partielle Vakuum aus das Monomer 16 im Behälter 18 und bewirkt, dass dieses aus dem Behälter 18 in den Mischraum 9 gesaugt wird, d.h. zu dem Polymer 17 darin, strömt (siehe Fig. 2). Die Wände 30 des Behälters 18 sind so ausgeführt (z.B. so flexibel und/oder elastisch), dass dieselben hierbei zusammengesaugt werden sodass sich der Behälter 18 vollständig oder nahezu vollständig entleert (siehe Fig. 3).

Nachdem Monomer 16 sich im Mischraum 9 in Kontakt mit dem Polymer 17 befindet, wird die Mischung durchgeführt, indem das nun zur Mischung verwendete Mischorgan 6 im Mischraum 9 hin und her geschoben und eventuell gedreht wird, bis sich das Monomer 16 und das Polymer 17 genügend gemischt haben (siehe Fig. 3). Bei dieser Mischung wird vorzugsweise partielles Vakuum im dem Mischraum 9 durch die vakuumerzeugende Vorrichtung 12 erzeugt.

Danach wird ein Festhalteorgan 33, das den Kolben 5 an einem Endteil des zylindrischen Behälters 3 festhält, entfernt (siehe Fig. 4), wodurch das partielle Vakuum im Mischraum 9 den Kolben 5 in den Behälter 3 hinein saugt. Hierdurch wird der Knochenzement 2 und an verschiedenen Stellen im Mischraum 9 festsitzende Knochenzementklumpen im Mischraum 9 bei dem Deckel 4 aufgesammelt.

Zum Entleeren des Mischraums 9 wird der Schlauch 13 zur vakuumerzeugenden Vorrichtung 12 vom Anschlussrohr 10 abgenommen und das Entleerrohr 14 an das Anschlussrohr 10 angeschlossen (siehe Fig. 5). Danach wird der Mischbehälter 1 in die Druckvorrichtung 15 gelegt und der Kolben 5 mit dieser in Richtung gegen den Deckel 4 gedrückt, wodurch der Knochenzement 2 durch das Entleerrohr 14 aus dem Mischraum 9 gedrückt wird. Der Kolben 5 wird mit einem Druckorgan 34 der Druckvorrichtung 15 verschoben, wobei die Behälterwände 30 zwischen dem Kolben 5 und dem Druckorgan 34 liegen. Die Behälterwände 30 können vorzugsweise in den hülsförmigen Teil 20 des Kolbens 5 platz nehmen und/oder in diesen hineingesaugt werden und hindern damit nicht dass das Druckorgan 34 in diesen hülsförmigen Teil 20 hineingeschoben wird (siehe Fig. 6).

Die Wände 30 des Behälters 18 können aus solchen Metallmaterial bestehen dass der Stoff 16 darin bei Strahlenwirkung, die bei der Bestrahlungssterilisierung auftritt, geschützt wird.

Sowohl der Behälter 18 als auch das Innere des Mischbehälters 1 können sterilisiert sein.

Die oben beschriebenen und in den Figuren gezeigten Ausführungen können im Rahmen der Patentansprüche variieren. Demnach kann beispielsweise der Mischbehälter 1 ein Mischbecher sein, von dem der darin fertiggestellte Knochenzement 2 in eine Kartusche gefüllt wird, um damit appliziert zu werden. Partielles Vakuum kann innerhalb aber auch ausserhalb der Grenzen 60 - 95 % liegen und eventuell schon im voraus im Mischbehälter 1 erzeugt sein.

Der Stoff 17 kann in einem in den Mischraum 9 verpackten Extrabehälter verpackt sein, der Kolben 5 und/oder der Behälter 18 kann/können andere Form/Formen aufweisen; der Behälter 18 kann jedoch in einer anderen Position als am Kolben 5 angeordnet sein. Der Öffner zum Öffnen der Wand 19 ist als Dorn 26 am Mischorgan 6 beschrieben und gezeigt, derselbe kann jedoch aus einem anderen Organ das andersartig angeordnet ist, bestehen.

### LITERATURÜBERSICHT

### Vakuummischung von Knochenzement

1. **A charactarization of Polymethylmethacrylate bone cement.** *Haas, S S, Braner G M, Dickson G. J Bone Joint Surg, 57-A, 380-391, 1975.*
2. **Strength of polymethylmethacrylate increased by vacuum mixing.** *Lidgren L, Drar H, Möller J. Acta Orthop Scand 55: 536-541, 1984.*
3 **Bone cement improved by vacuum mixing and chilling.** *Lidgren L, Bodelind B, Möller J. Acta Orthop Scand 57: 27-32, 1987.*
4. **High vacuum as a method of reducing porosity of polymethlmethacrylate.** *Alkire M JH, Dabezies E J, Hastings P R. Orthopedics 10-11: 1533-1539, 1987.*
5. **Vacuum mixing of acrylic bone cement** *Wixson R L, Lautenschlager E P, Novak M A,. J Arthroplasty 2: 141-149, 1987.*
6. **Effects of preparation techniques on the porosity of acrylic cements**. *Schreurs B W, Spierings T J, Huiskes R, Slooff J J H. Acta Orthop Scand 59(4): 403-409, 1988.*
7. **Efficiency of bone cement mixing Systems - A gas chromatographic study.** *Darre E, Vedel P, Steen Jensen J. Adv Orthop Surg 12: 106-108, 1988.*
8. **Porosity in manually mixed bone cement.** *Linden U, Clin Orthop 231: 110-112, 1988.*
9. **Air inclusion in bone cement.** *Linden U, Gillquist J. Clin Orthop 247: 148-151, 1989.*
10. **Fatigue properties of bone cement.** *Linden U, Acta Orthop Scand 60: 431-433, 1989.*
11. **A fractographic analysis of in vivo poly(methylmethacrylate) bone cement failure mechanism.** *Topoleski LDT, Ducheyne P, Cuckler JM. J Biomedical Materials Res. 24: 135-54, 1990.*
12. **Comparison of the diametral shrinkage of centrifuged and uncentrifuged.** *Davies J P, O'Connor D O, Burke D W, Harris W H. The 16th Annual Meeting of the Society for Biomaterials, 23, 1990.*
13. **Effect of pore size and morphology on fatigue crack initiation in acrylic bone cements.** *Gilbert J L, Menis D W, Smith S M, Lautenschlager E P, Wixson R W. The 16th Annual Meeting of the Society for Biomaterials, 103, 1990.*
14. **Effect of vacuum mixing on interfacial bone cement fracture toughness.** *Menis D L, Wixson R L, Gilbert J L, Lautenschlager E P. The 16th Annual Meeting of the Society for Biomaterials, 67, 1990.*
15. **Effect of vacuum mixing on the mechanical properties of antibiotic-impregnated polymethylmethacrylate bone cement.** *Askew M J: Kufel M F, Fleissner P R Jr., GradisarI A Jr., Salstrom S-A, Ton J S. J Biomed Mater Res 24: 573-580, 1990.*
16. **Optimization and comparison of three vacuum mixing systems for porosity reduction of Simplex P cement.** *Davies J P, Harris W H. Clin Orthop 254: 261-269, 1990*
17. **Porosity of various preparations of acrylic bone cements**. *Jasty M, Davies J, O'Connor D, Burke D, Harrigan T, Harris W. Clin Orthop 259: 122-129, 1990.*
18. **The effects of the mixing techniques of Gentamicin-loaded bone cement on the release of antibiotics.** *Wahling, H, Dingeldein E. Orthop Rel Sci. 1: 121-124, 1990.*
19. **A fractographic investigation of PMMA bone cement focusing on the relationship between porosity reduction and increased fatigue life.** *Jahres SP, Jasty M*, *Davies J, Piehler. J Biomed Mater Res 26: 651-62, 1992.*
20. **Borderline indications for use of cement in total joint replacements.** *Jasty M, Mulroy R, Harris W H. Chir Organi Mov 77(4): 397-404, 1992.*
21. **Do we need to vacuum mix or centrifuge cement?** *Wixson R L. Clin Orthp 285: 84-90, 1992.*
22. **Influence of vacuum mixing on mechanical properties of homemade bone cement.** *Chen XX. Chung-Hua -Wai -Ko -Tsa -Chih, Oct, Vol: 30(10), 593-5, 635-6, 1992.*
**23. Observation on PMMA bone cement with scanning electron-microscope:the efficacity of vacuum-mixing**.*Chen H-X, Lu H-S, Lin J-H. Arthritis Clinic & Research Center, People's Hospital, Beijing Medical University, Beijing, P.R. China. SIROT 93 Seoul VI World Congress, Korea, August 27-30, 1993.*
24. **Porosity of bone cement reduced by mixing and collecting under vacuum.** *Wang J-S, Franzén H, Jonsson E, Lidgren L. Acta Orthop Scand 64 (2): 143-146, 1993.*
**25. The effects of vacuum mixing on the microscopic homogenicity of palacos R bone cement.** *Wand J-S, Goodman S B, Franzén H, Aspenberg P, Lidgren L. Eur J Exp Musculoskeletal Res 2: 000-000 , 1993.*

## Patentansprüche

1. Mischvorrichtung zum Mischen von zumindest zwei mischbaren Stoffen zum Herstellen von Knochenzement,
wobei die zwei Stoffe in einem Mischraum (9) in einem Mischbehälter (1) gemischt werden,
wobei beide Stoffe voneinander getrennt in je einem Behälter (1,18) angeordnet sind,
wobei der Mischbehälter einen Kolben (5) aufweist, der mit einer Druckvorrichtung verschoben werden kann um den Knochenzement aus dem Mischraum zu drücken,
wobei der Kolben in einer Ausgangstellung an einem ersten Ende des Mischbehälters angeordnet ist,
wobei ein Anschluss für eine vakuumerzeugende Vorrichtung (12) an einem zweiten, dem ersten Ende entgegengesetzten Ende des Mischbehälters angeordnet ist, welche vakuumerzeugende Vorrichtung vorgesehen ist, Vakuum im Mischraum zu erzeugen, und
wobei ein Mischorgan (6) im Mischraum (9) angeordnet ist, um die beiden Stoffe (16, 17) im Mischraum (9) zu mischen,
**dadurch gekennzeichnet,**
dass der eine Behälter (18) mit dem einen Stoff (16) flexibel ausgebildet ist,
dass der flexible Behälter (18) an dem ersten Ende des Mischbehälters (1) angeordnet ist,
dass ein Öffner (26) vorgesehen ist, um zumindest eine Verbindung (27) zwischen den getrennt angeordneten Stoffen (16, 17) herzustellen, um diese miteinander in Kontakt zu bringen und mischen zu können, und
dass die vakuumerzeugende Vorrichtung (12) während des Öffnens und/oder nach dem Öffnen der Verbindung (27) zwischen den Stoffen (16, 17) im Mischraum (9) Vakuum erzeugt, um den einen Stoff (16) durch die Verbindung (27) zu dem anderen Stoff (17) zu saugen.

2. Mischvorrichtung nach Anspruch 1, **dadurch gekennzeichnet,** dass die zwei Stoffe aus flüssigem Monomer (16) und pulverförmigem Polymer (17) bestehen.

3. Mischvorrichtung nach einem der vorhergehenden Ansprüche, **dadurch gekennzeichnet,** dass das Mischorgan (6) an dem zweiten Ende des Mischbehälters (1) gelagert ist.

4. Mischvorrichtung nach einem der vorhergehenden Ansprüche, **dadurch gekennzeichnet,** dass der Öffner (26) von dem Mischorgan (6) gebildet wird.

5. Mischvorrichtung nach Anspruch 4, **dadurch gekennzeichnet,** dass der Öffner aus zumindest einem Dorn (26) am Mischorgan (6) besteht.

6. Mischvorrichtung nach einem der vorhergehenden Ansprüche, **dadurch gekennzeichnet**, dass zumindest der eine Stoff (17), vorzugsweise pulverförmiges Polymer, in einem Mischraum (9) auf der einen Seite des Kolbens (5) angeordnet ist.

7. Mischvorrichtung nach Anspruch 6, **dadurch gekennzeichnet**, dass der andere Stoff (16), vorzugsweise flüssiges Monomer, in einem Behälter (18) auf der anderen Seite des Kolbens (5) angeordnet ist und dass die Verbindung (27) zwischen den getrennt angeordneten Stoffen (16, 17) durch Öffnung des Kolbens (5) mit dem Öffner (26) erfolgt.

8. Mischvorrichtung nach Anspruch 7, **dadurch gekennzeichnet**, dass einer der Stoffe (16), vorzugsweise flüssiges Monomer, in einem äusseren Raum ausserhalb des Kolbens (5) in einem Behälter (18) angeordnet ist.

9. Mischvorrichtung nach einem der Ansprüche 6-8, **dadurch gekennzeichnet,** dass der Behälter (18) mit dem einen Stoff (16), vorzugsweise flüssiges Monomer, am Kolben (5) befestigt ist, dass der Kolben (5) eine Wand (19) des Behälters (18) bildet, welche Wand (19) den Stoff (16), vorzugsweise flüssiges Monomer, im Behälter (18) vom Stoff (17), vorzugsweise pulverförmiges Polymer, im Mischraum (9) trennt, dass der Öffner (26), der vorzugsweise am Mischorgan (6) angeordnet ist, die vom Kolben (5) gebildete Wand (19) des Behälters (18) zu öffnen und dadurch eine Verbindung (27) zwischen dem Behälter (18) und dem Mischraum (9) zu bilden und dass im Mischraum (9) Vakuum erzeugt wird wodurch der Stoff (16), vorzugsweise flüssiges Monomer, aus dem Behälter (18) durch die Verbindung (27) zu dem Stoff (17), vorzugsweise pulverförmiges Polymer, im Mischraum (9) gesaugt wird.

10. Mischvorrichtung nach Anspruch 9, **dadurch gekennzeichnet,** dass der am Kolben (5) angeordnete Behälter (18) vollständig oder zumindest hauptsächlich in den Mischraum (9) entleert wird.

11. Mischvorrichtung nach Anspruch 9 oder 10, **dadurch gekennzeichnet,** dass ein hülsenförmiger Endteil (29) des Behälters (18) an einem hülsenförmigen Teil (20) des Kolbens (5) angeordnet ist.

12. Mischvorrichtung nach Anspruch 11, **dadurch gekennzeichnet,** dass der hülsenförmige Endteil (29) des Behälters (18) den hülsenförmigen Teil (20) des Kolbens (5) umgibt und dichtend an dessen Aussenseite (21) angeordnet ist, wobei vorzugsweise der hülsenförmige Teil (20) des Kolbens (5) mit einer Aussenseite (21) gegen eine Innenseite (22) des zylindrischen Behälters (3) anliegt und an der Aussenseite (21) eine Nute (28) aufweist in der der hülsenförmige Endteil (29) des Behälters (18) eingreift.

13. Mischvorrichtung nach einem der Ansprüche 9-12, **dadurch gekennzeichnet,** dass der Kolben (5) und der Behälter (18) dadurch mit einander verbunden sind indem dieselben durch Aufwärmung mit einander verschmolzen sind.

14. Mischvorrichtung nach Anspruch 13, **dadurch gekennzeichnet,** dass der Behälter mehrere Schichten (31, 32) aufweist von denen zumindest eine gegen den Kolben (5) anliegende innere Schicht (31) aus denselben oder ähnlichen Kunststoff wie der Kolben (5) oder Befestigungsteile des Kolbens (5) für den Behälter (18) besteht, wobei die Kunststoffteile des Kolbens (5) und des Behälters (18) durch Aufwärmung mit einander verschmolzen werden können und dass zumindest eine andere Schicht des Behälters (18), vorzugsweise eine äussere Schicht (32), aus Metallmaterial, vorzugsweise Aluminiummaterial, besteht.

15. Mischvorrichtung nach einem der Ansprüche 9-14, **dadurch gekennzeichnet,** dass durch das im Mischraum (9) erzeugte Vakuum nach Öffnung der Verbindung (27) mit dem am Kolben (5) angeordneten Behälter (18) ein Vakuum in dem Behälter (18) entsteht, wodurch am Kolben (5) angeordnete Wände (30) des Behälters (18) vollständig oder zumindest wesentlich zusammengesaugt werden.

16. Mischvorrichtung nach Anspruch 15, **dadurch gekennzeichnet,** dass die am Kolben (5) angeordneten Wände (30) des Behälters (18) vollständig oder zumindest teilweise aus flexiblen Material bestehen und/oder solche flexible Eigenschaften aufweisen dass die Wände (30) vollständig oder zumindest teilweise in einen hülsförmigen Teil (20) des Kolbens (5) hineingesaugt werden können.

17. Mischvorrichtung nach einem der Ansprüche 9-16, **dadurch gekennzeichnet,** dass sich die Wände (30) des Behälters (18) nach der Entleerung desselben beim Verschieben des Kolbens (5) mit der Druckvorrichtung (15) zur Entleerung des Mischraums (9) zumindest teilweise zwischen dem Kolben (5) und einem Druckorgan (34) der Druckvorrichtung (15) befinden.

18. Mischvorrichtung nach einem der vorhergehenden Ansprüche, **dadurch gekennzeichnet**, dass der Mischbehälter (1) am Platz, an dem die Mischung durchgeführt wird, an die vakuumerzeugende Vorrichtung (12) angeschlossen wird, um partielles Vakuum in dem Mischraum (9) zu erzeugen.

19. Mischvorrichtung nach einem der vorhergehenden Ansprüche, **dadurch gekennzeichnet,** dass das erzeugte Vakuum während der Aufsammlung von Knochenzementklumpen, die im Mischraum (9) aufgesammelt werden, beibehalten wird.

20. Mischvorrichtung nach einem der vorhergehenden Ansprüche, **dadurch gekennzeichnet,** dass der Mischbehälter (1) und/oder die zu mischenden Stoffe (16, 17) steril verpackt sind.

21. Mischvorrichtung nach einem der vorhergehenden Ansprüche, **dadurch gekennzeichnet,** dass zusätzlich in zumindest einem der Behälter (1, 18) antibakterielle Wirkstoffe angeordnet sind.

22. Mischvorrichtung nach einem der vorhergehenden Ansprüche, **dadurch gekennzeichnet,** dass zusätzlich in zumindest einem der Behälter (1, 18) knochenwachstumsfördernde Substanzen angeordnet sind.

23. Mischvorrichtung nach einem der vorhergehenden Ansprüche, **dadurch gekennzeichnet,** dass zumindest ein Behälter (18) mit dem einen Stoff (16), vorzugsweise flüssigen Monomer, aus einem Metallmaterial, beispielsweise Aluminium, besteht, um den Stoff (16), vorzugsweise Monomer, in dem Behälter (18) bei Strahlenwirkung, die bei Bestrahlungssterilisierung auftritt, zu schützen.

## Claims

1. Mixing device for mixing at least two substances for manufacturing bone cement,
wherein the two substances (16, 17) are mixed in a mixing space (9) in a mixing container (1),
wherein both substances are provided separated from each other in a container (1, 18) for each substance,
wherein the mixing container comprises a piston (5) which may be displaced by a pressure device for pressing the bone cement out of the mixing space,
wherein the piston in a starting position is provided at a first end of the mixing container,
wherein a connection for a vacuum generating device (12) is located at a second end, opposite so said first end, of the mixing container, said vacuum generating device being provided to generate a vacuum in the mixing space, and
wherein a mixing means (6) is provided in the mixing space (9) for mixing the two substances (16, 17) in said mixing space (9),
**characterized in**
that one of the containers (18) with one of the substances (16) is flexible,
that the flexible container (18) is provided at said first end of the mixing container (1),
that an opener (26) is provided for establishing a connection (27) between the separately provided substances (16, 17) for bringing said substances in contact with each other for mixing, and
that the vacuum generating device (12) during opening and/or after opening of the connection (27) between the substances (16, 17) generates a vacuum in the mixing space (9) for sucking one of the substances (16) through the connection (27) to the other substance (17).

2. Mixing device according to claim 1, **characterized in** that the two substances consist of a liquid monomer (16) and a pulverulent polymer (17).

3. Mixing device according to any preceding claim, **characterized in** that the mixing means (6) is mounted at said second end of the mixing container (1).

4. Mixing device according to any preceding claim, **characterized in** that the opener (26) is defined by the mixing means (6).

5. Mixing device according to claim 4, **characterized in** that the opener (26) consists of at least one mandrel (26) on the mixing means (6).

6. Mixing device according to any preceding claim, **characterized in** that at least one of the substances (17) preferably the pulverulent polymer, is provided in a mixing space (9) on one side of the piston (5).

7. Mixing device according to claim 6, **characterized in** that the other substance (16), preferably liquid monomer, is located in a container (18) on the other side of the piston (5) and that the connection (27) between the separately provided substances (16, 17) is obtained by opening the piston (5) by means of the opener (26).

8. Mixing device according to claim 7, **characterized in** that one of the substances (16), preferably liquid monomer, is provided in a container (18) in an outer space outside the piston (5).

9. Mixing device according to any of claims 6 - 8, **characterized in** that the container (18) with one of the substances (16), preferably liquid monomer, is attached to the piston (5), that the piston (5) defines a wall (19) of the container (18) which wall separates said one substance (16), preferably liquid monomer, in the container (18) from the substance (17), preferably pulverulent polymer, in the mixing space (9), that the opener (26) preferably is located on the mixing means (6) for opening the wall (19) of the container (18) defined by the piston (5) and thereby establish a connection (27) between the container (18) and the mixing space (9), and that vacuum is generated in the mixing space (9), whereby said one substance (16), preferably liquid monomer, is sucked out of the container (18) and through the connection (27) to the substance (17), preferably pulverulent polymer, in the mixing space (9).

10. Mixing device according to claim 9, **characterized in** that the container (18) provided on the piston (5) is completely or at least substantially emptied into the mixing space (9).

11. Mixing device according to claim 9 or 10, **characterized in** that a sleeve-like end portion (29) of the container (18) is provided on a sleeve-like member (20) of the piston (5).

12. Mixing device according to claim 11, **characterized in** that the sleeve-like end portion (29) of the container (18) surrounds the sleeve-like member (20) of the piston (5) and sealingly engages the outer side (21) thereof, whereby preferably the sleeve-like member (20) of the piston (5) with an outer side (21) engages an inner side (22) of the cylindrical container (3) and whereby said member on said outer side (21) is provided with a groove (28) wherein the sleeve-like end portion (29) of the container (18) is inserted.

13. Mixing device according to any of claims 9 - 12, **characterized in** that the piston (5) and the container (18) are connected with each other being confounded with each other through heating.

14. Mixing device according to claim 13, **characterized in** that the container is made up of several layers (31, 32) of which at least one inner layer (31) engaging the piston (5) consists of the same or similar plastic as the piston (5) or as mounting members of said piston for the container (18), whereby the plastic members of the piston (5) and container (18) can be confounded with each other by heating, and that at least another layer (32) of the container (18), preferably an outer layer (32), consists of a metallic material, preferably aluminium.

15. Mixing device according to any of claims 9 - 14, **characterized in** that because of the vacuum generated in the mixing space (9), a vacuum is generated also in the container (18) after opening of the connection (27) with the container (18) located on the piston (5), whereby walls (30) of the container (18) provided on the piston (5) are completely or at least substantially drawn together.

16. Mixing device according to claim 15, **characterized in** that the walls (30) of the container (18) located on the piston (5) consist completely or partially of flexible material and/or have such flexible properties that they can be completely or at least partially sucked into a sleeve-like member (20) of the piston (5).

17. Mixing device according to any of claims 9 - 16, **characterized in** that the walls (30) of the container (18), after emptying thereof by displacement of the piston (5) by means of the pressure device (15), for emptying the mixing space (9) are at least partially located between the piston (5) and a pressure means (34) of the pressure device (15).

18. Mixing device according to any preceding claim, **characterized in** that the mixing container (1), at the spot where mixing is carried out, is connected with the vacuum generating device (12) for generating partial vacuum in the mixing space (9).

19. Mixing device according to any preceding claim, **characterized in** that the generated vacuum is maintained during collection of clumps of bone cement which are collected in the mixing space (9) .

20. Mixing device according to any preceding claim, **characterized in** that the mixing container (1) and/or the substances (16, 17) to be mixed are packed up in sterile condition.

21. Mixing device according to any preceding claim, **characterized in** that antibacterial active substances are also provided in at least one of the containers (1, 18).

22. Mixing device according to any preceding claim, **characterized in** that bone growth promoting substances are also provided in at least one of the containers (1, 18).

23. Mixing device according to any preceding claim, **characterized in** that at least one container (18) with one of the substances (16), preferably liquid monomer, is made of a metallic material, preferably aluminium, for protecting said substance, preferably monomer, in the container (18) against the radiation effect occurring during sterilization by radiation.

## Revendications

1. Dispositif de mélange pour mélanger au moins deux substances miscibles pour la préparation d'un ciment pour ostéosynthèse,
dans lequel les deux substances sont mélangées dans une chambre de mélange (9) dans un récipient de mélange (1),
dans lequel les deux substances sont disposées en étant séparées l'une de l'autre dans respectivement un récipient (1, 18),
dans lequel le récipient de mélange présente un piston (5) qui peut être déplacé à l'aide d'un dispositif de pression pour presser le ciment pour ostéosynthèse hors de la chambre de mélange,
dans lequel le piston est disposé dans une position de départ contre une première extrémité du récipient de mélange,
dans lequel on dispose un raccord pour un dispositif (12) générant du vide à une seconde extrémité du récipient de mélange opposée à la première extrémité, ledit dispositif générant du vide étant prévu pour générer du vide dans la chambre de mélange, et
dans lequel un organe de mélange (6) est disposé dans la chambre de mélange (9) pour mélanger les deux substances (16, 17) dans la chambre de mélange (9),
caractérisé en ce que
le premier récipient (18) comprenant la première substance (16) est réalisé pour le rendre flexible,
en ce que le récipient flexible (18) est disposé contre la première extrémité du récipient de mélange (1),
en ce qu'on prévoit un dispositif d'ouverture (26) pour établir au moins une liaison (27) entre les substances (16, 17) disposées séparément pour pouvoir amener ces dernières en contact l'une avec l'autre et pour pouvoir les mélanger,
en ce que le dispositif (12) générant du vide génère du vide dans la chambre de mélange (9) au cours de l'ouverture et après l'ouverture de la liaison (27) entre les substances (16, 17) pour aspirer la première substance (16) à travers la liaison (27) en direction de l'autre substance (17).

2. Dispositif de mélange selon la revendication 1, caractérisé en ce que les deux substances sont constituées par un monomère liquide (16) et par un polymère pulvérulent (17).

3. Dispositif de mélange selon l'une quelconque des revendications précédentes, caractérisé en ce que l'organe de mélange (6) est monté contre la deuxième extrémité du récipient de mélange (1).

4. Dispositif de mélange selon l'une quelconque des revendications précédentes, caractérisé en ce que le dispositif d'ouverture (26) est formé par l'organe de mélange (6).

5. Dispositif de mélange selon la revendication 4, caractérisé en ce que le dispositif d'ouverture est constitué par au moins une épine (26) montée sur l'organe de mélange (6).

6. Dispositif de mélange selon l'une quelconque des revendications précédentes, caractérisé en ce qu'au moins la première substance (17), de préférence du polymère pulvérulent, est disposée dans une chambre de mélange (9) d'un côté du piston (5).

7. Dispositif de mélange selon la revendication 6, caractérisé en ce que l'autre substance (16), de préférence un monomère liquide, est disposée dans un récipient (18) de l'autre côté du piston (5) et en ce que la liaison (27) entre les substances (16, 17) disposées séparément a lieu par ouverture du piston (5) avec le dispositif d'ouverture (26).

8. Dispositif de mélange selon la revendication 7, caractérisé en ce qu'une des substances (16), de préférence le monomère liquide, est disposée dans une chambre externe à l'extérieur du piston (5) dans un récipient (18).

9. Dispositif de mélange selon l'une quelconque des revendications 6 à 8, caractérisé en ce que le récipient (18) comprenant la première substance (16), de préférence un monomère liquide, est fixé au piston (5), en ce que le piston (5) forme une paroi (19) du récipient (18), ladite paroi (19) séparant la substance (16), de préférence un monomère liquide, dans le récipient (18), de la substance (17), de préférence un polymère pulvérulent, dans la chambre de mélange (9), en ce que le dispositif d'ouverture (26), qui est disposé de préférence contre l'organe de mélange (6), ouvre la paroi (9) du récipient (18) formée par le piston (5) et forme ainsi une liaison (27) entre le récipient (18) et la chambre de mélange, et en ce qu'un vide est généré dans la chambre de mélange (9), par lequel la substance (16), de préférence un monomère liquide, est aspirée hors du récipient (10) à travers la liaison (27) en direction de la substance (17), de préférence un polymère pulvérulent, dans la chambre de mélange (9).

10. Dispositif de mélange selon la revendication 9, caractérisé en ce que le récipient (18) disposé contre le piston (5) est vidé complètement ou au moins principalement dans la chambre de mélange (9).

11. Dispositif de mélange selon la revendication 9 ou 10, caractérisé en ce qu'une partie terminale (29) du récipient (18) en forme de manchon est disposée contre une partie (20) du piston (5) en forme de manchon.

12. Dispositif de mélange selon la revendication 11, caractérisé en ce que la partie terminale (29) du récipient (18) en forme de manchon entoure la partie (20) du piston (5) en forme de manchon et est disposée en étanchéité contre le côté externe (21) de ce dernier, dans lequel de préférence la partie (20) du piston (5) en forme de manchon vient s'appuyer avec son côté externe (21) contre le côté interne (22) du récipient cylindrique (3) et présente une rainure (28) sur le côté externe (21) dans laquelle vient s'insérer la partie terminale (29) du récipient (18) en forme de manchon.

13. Dispositif de mélange selon l'une quelconque des revendications 9 à 12, caractérisé en ce que le piston (5) et le récipient (18) sont reliés l'un à l'autre en les portant à fusion l'un avec l'autre par chauffage.

14. Dispositif de mélange selon la revendication 13, caractérisé en ce que le récipient présente plusieurs couches (31, 32) parmi lesquelles au moins une couche interne (31) disposée contre le piston (5) est constituée de la même substance synthétique que celle du piston (5) ou que celle d'éléments de fixage du piston (5) pour le récipient (18) ou d'une substance synthétique analogue, dans lequel les parties du piston (5) et du récipient (18) en matière synthétique peuvent être portées à fusion l'une avec l'autre par chauffage et en ce qu'au moins une autre couche du récipient (18), de préférence une couche externe (32), est constituée d'une matière métallique, de préférence d'une matière en aluminium.

15. Dispositif de mélange selon l'une quelconque des revendications 9 à 14, caractérisé en ce que, grâce au vide généré dans la chambre de mélange (9), après l'ouverture de la liaison (27) avec le récipient (18) disposé contre le piston (5), on obtient un vide dans le récipient (18), par lequel les parois (30) du récipient (18) disposées contre le piston (5) sont aspirées de concert complètement ou au moins de manière essentielle.

16. Dispositif de mélange selon la revendication 15, caractérisé en ce que les parois (30) du récipient (18) disposées contre le piston (5) sont constituées complètement ou au moins en partie d'une matière flexible et/ou présentent des propriétés flexibles telles que les parois (30) sont à même d'être aspirées complètement ou au moins en partie dans une partie (20) du piston (5) en forme de manchon.

17. Dispositif de mélange selon l'une quelconque des revendications 9 à 16, caractérisé en ce que les parois (30) du récipient (18), après avoir fait le vide en déplaçant le piston (5) avec le dispositif de pression (15) pour vider la chambre de mélange (9), se trouvent au moins en partie entre le piston (5) et un organe de pression (34) du dispositif de pression (15).

18. Dispositif de mélange selon l'une quelconque des revendications précédentes, caractérisé en ce que le récipient de mélange (1), à l'endroit où l'on procède au mélange, est raccordé au dispositif (12) générant un vide pour générer un vide partiel dans la chambre de mélange (9).

19. Dispositif de mélange selon l'une quelconque des revendications précédentes, caractérisé en ce que le vide généré est maintenu au cours du rassemblement des morceaux de ciment pour ostéosynthèse qui sont rassemblés dans la chambre de mélange (9).

20. Dispositif de mélange selon l'une quelconque des revendications précédentes, caractérisé en ce que le récipient de mélange (1) et/ou les substances à mélanger (16, 17) sont emballés dans des conditions stériles.

21. Dispositif de mélange selon l'une quelconque des revendications précédentes, caractérisé en ce que des substances à activité antibactérienne sont disposées en outre dans au moins un des récipients (1, 18).

22. Dispositif de mélange selon l'une quelconque des revendications précédentes, caractérisé en ce que des substances favorisant la croissance des os sont disposées en outre dans au moins un des récipients (1, 18).

23. Dispositif de mélange selon l'une quelconque des revendications précédentes, caractérisé en ce qu'au moins un récipient (18) comprenant la première substance (16), de préférence un monomère liquide, est constitué d'une matière métallique, par exemple d'aluminium, pour protéger la substance (16), de préférence un monomère dans le récipient (18) lors de l'effet de rayonnement qui se produit lors de la stérilisation par exposition à un rayonnement.
